# EUROPÄISCHE PATENTSCHRIFT

(11) **EP 1 962 732 B1**
(45) Veröffentlichungstag und Bekanntmachung des Hinweises auf die Patenterteilung: **19.08.2009**
(21) Anmeldenummer: 06828624.4
(22) Anmeldetag: 12.12.2006
(51) Int. Cl.: A61F 2/58, B25J 15/08

(54) **FINGERPROTHESE SOWIE HANDPROTHESE**
FINGER PROSTHESIS AND HAND PROSTHESIS
PROTHESE DE DOIGT ET PROTHESE DE MAIN

(30) Priorität: 20.12.2005 DE 102005061265
(43) Veröffentlichungstag der Anmeldung: 03.09.2008
(73) Patentinhaber: Otto Bock HealthCare GmbH, 37115 Duderstadt (DE)
(72) Erfinder: PUCHHAMMER, Gregor, 1130 Wien (AT); KUSCHNIGG, Peter, 2731 Neusiedl (AT)
(74) Vertreter: Stornebel, Kai
(86) Internationale Anmeldenummer: PCT/DE2006/002174
(87) Internationale Veröffentlichungsnummer: WO 2007/076762

(56) Entgegenhaltungen:
- EP-A1- 1 375 087
- FR-A1- 2 822 404
- US-A- 2 567 066
- US-A- 4 685 924
- US-A1- 2005 043 822

## Beschreibung

Die Erfindung betrifft eine Fingerprothese mit einem Grundelement, das zur Aufnahme von Kräften und Momenten ausgebildet und mit einem elastischen Überzug zur Erzielung einer natürlichen Optik versehbar ist. Weiterhin betrifft die Erfindung eine Handprothese mit einem Chassis und einer daran angeordneten Fingerprothese.

Aus dem Stand der Technik sind Finger- und Handprothesen bekannt, bei denen die Fingerprothesen einen metallischen Kern aufweisen, der entweder einstückig ausgebildet oder aus mehreren, den Fingergliedern entsprechenden Komponenten, die gelenkig miteinander verbunden sind, aufgebaut ist. Die Fingerprothesen sind gelenkig an dem Chassis angeordnet und können über Aktuatoren bewegt werden, so dass eine Greifbewegung nachgebildet werden kann. Ein Beispiel für einen einteiligen Aufbau einer Fingerprothese ist in der US 2004/0015240 A1 beschrieben.

Mechanisch komplexere Fingerprothesen sehen eine Krümmung der Fingerprothese in zumindest einem weiteren Fingergelenk zusätzlich zu der Verschwenkung um das Fingergrundgelenk vor. Eine solche Ausgestaltung ist beispielsweise in der US 2005/0021154 A1 beschrieben.

Um der Finger- oder Handprothese eine möglichst natürliche Anmutung zu geben, wird über den mechanischen Aufbau ein Gummiüberzug in Handform gestülpt, der neben einer Schutzfunktion für die mechanischen Komponenten eine Polsterung und die einer natürlichen Hand angenäherte optische Anmutung bereitstellt. Eine solche Prothese wird in der US 4,685,924 offenbart. Ein solcher Überzug ist aufwendig herzustellen, schwer über die mechanischen Komponenten überzuziehen und hat nur eine retativ schwache haptische Entsprechung mit einer natürlichen Hand.

Aufgabe der vorliegenden Erfindung ist es, eine Fingerprothese und eine Handprothese bereitzustellen, die eine verbesserte natürliche Anmutung aufweisen, preiswerter herzustellen und leichter zu nutzen sind.

Erfindungsgemäß wird diese Aufgabe mit einer Fingerprothese mit den Merkmalen des Anspruchs 1 sowie mit einer Handprothese mit den Merkmalen des Anspruchs 13 gelöst. Vorteilhafte Ausgestaltungen und Weiterbildungen der Erfindung sind in den Unteransprühen beschrieben.

Die erfindungsgemäße Fingerprothese mit einem Grundgelenk, das zur Aufnahme von Kräften und Momenten ausgebildet und mit einem elastischen Überzug zur Erzielung einer natürlichen Optik versehbar ist, sieht vor, dass das Grundgelenk als ein formstabiler Kern ausgebildet ist, der an mit Weichteilstellen an natürlichen Fingern korrespondierenden Stellen Ausnehmungen aufweist, die mit einem Elastomerwerkstoff unter Bildung einer natürlichen Fingerkontur aufgefüllt sind. Neben einem harten Grundelement oder Kern werden somit Bereiche hoher Elastizität bereitgestellt, beispielsweise im Bereich der Fingerspitzen oder der zur Handinnenfläche zeigenden Flächen der Fingerglieder, um eine möglichst natürliche Haptik bereitzustellen. Bei motorisch angetriebenen Fingerprothesen wird dadurch ein verbessertes Greifen ermöglicht, da der Kontakt zu den gegriffenen Gegenständen wie bei einer natürlichen Hand an elastisch verformbaren Bereichen stattfindet, wodurch sich neben einer erhöhten Griffsicherheit auch eine natürlichere Greifbewegung ergibt.

Eine Weiterbildung der Erfindung sieht vor, dass das Grundelement als ein Hohlkörper ausgebildet ist, um die gesamte Fingerkonstruktion leicht zu gestalten. Bevorzugt ist das Grundelement aus einem Kunststoff, insbesondere einem Thermoplasten oder aus einem Verbundwerkstoff aufgebaut, um das Gewicht der Fingerprothese weiter zu verringern, ohne Einschränkungen hinsichtlich der Festigkeit in Kauf nehmen zu müssen. Alternativ kann das Grundelement auch aus einem Metallwerkstoff, insbesondere Leichtmetall oder einer Metalllegierung ausgebildet sein. Eine Kombination von Kunststoff und Metall kann ebenfalls eingesetzt werden.

Bevorzugt werden das Grundelement und der Elastomerwerkstoff in einem Zweikomponenten-Spritzgießverfahren gemeinsam verarbeitet, so dass die Fingerprothese bereits die endgültige Kontur bzw. Form und gleichzeitig die funktionellen Eigenschaften der an der Prothese angeordneten Fingerprothese aufweist. Durch eine entsprechende Farbgestaltung ist es möglich, die Fingerprothese einem natürlichen Finger angenähert auszugestalten, wobei an der Fingerprothese an denjenigen Stellen, die bei einem natürlichen Finger nachgiebig sind, entsprechend weiche Bereiche ausgebildet sind. Dies vereinfacht und verbilligt das Herstellverfahren von Fingerprothesen.

Eine Weiterbildung sieht vor, dass das Grundelement eine durchbrochene Struktur aufweist, so dass sich der Elastomerwerkstoff durch das Grundelement hindurch erstrecken kann. Das Grundelement ist dabei zumindest bereichsweise vollständig in dem Elastomerwerkstoff eingebettet, wodurch sich bei einer entsprechenden Materialwahl des Elastomerwerkstoffes eine hohe Elastizität und ein großer Verformungsweg bereitstellen lassen.

In dem Grundelement sowie alternativ oder ergänzend in dem Elastomerwerkstoff können Kanäle oder Aufnahmebereiche für Kabel oder Sensoren ausgebildet sein, beispielsweise um eine Rückkopplung über die Lage der einzelnen Fingerglieder zueinander und dadurch über das Maß der Fingerkrümmung zu erreichen.

An dem Grundelement kann eine Lagerung für eine Gelenkachse zur Befestigung an einem Chassis einer Prothese ausgebildet sein, wobei diese Lagerung elastisch ausgebildet sein kann. Bevorzugt lässt die Lagerung eine Verlagerung senkrecht zu der Drehachse zu, um eine der natürlichen Beweglichkeit der Finger angenäherte Anmutung zu erzielen. Durch eine solche "weiche" Lagerung der Fingerprothese ist eine seitliche Verlagerbarkeit und zusätzliche Nachgiebigkeit der Fingerprothese möglich, wobei sich eine starre Drehachse in einer weichen Aufnahme oder weichen Lagerung befindet. Dadurch wird zusätzlich der Eindruck einer starren, unnatürlichen, technischen Mechanik verringert und eine natürliche Anmutung des Greifens erreicht. Um die sehr hohen Belastungen der Fingerprothese und der Lagerung aufnehmen zu können, die bis zu 50 Nm betragen können, sind im Bereich des Fingergrundgelenkes Einlegeteile aus Metall zur Verstärkung der Lagerung angeordnet.

Zur Ermöglichung einer Rückkopplung der aufgebrachten Griffkraft ist es vorgesehen, dass im Bereich der Fingerspitze ein Sensor angeordnet, insbesondere in den Elastomerwerkstoff eingespritzt, eingedrückt oder eingeklebt ist. Der Sensor kann als Griffkraft-, Temperatur-, Rutsch- oder Beschleunigungssensor ausgebildet sein.

Um eine weitere Annäherung an einen natürlichen Finger zu erzielen, ist über das Grundelement und den Elastomerwerkstoff ein auswechselbarer Kunststoffüberzug in einer Hautgestaltung angeordnet, der an Farbe und Struktur einem natürlichen Finger angeglichen ist. Dieser Kunststoffüberzug kann sehr dünn ausgestaltet sein, wodurch er sehr preiswert herstellbar ist. Der Kunststoffüberzug kann sehr leicht über die Fingerprothese oder die gesamte Handprothese gezogen werden und überdeckt insbesondere die gelenkig miteinander verbundenen Bereiche. Im Falle einer Beschädigung oder irreversiblen Verschmutzung des Kunststoffüberzuges kann dieser leicht auch von dem Prothesennutzer wie ein Handschuh ausgewechselt werden.

Die erfindungsgemäße Handprothese ist mit einem Chassis und zumindest einer Fingerprothese versehen, wie oben beschrieben wurde. Die Fingerprothese ist bevorzugt gelenkig und/oder elastisch an dem Chassis gelagert, um über eine weiche Lagerung eine natürliche Anmutung zu erreichen.

Nachfolgend wird ein Ausführungsbeispiel der Erfindung anhand der beigefügten Figuren näher erläutert. Es zeigen:
- Figur 1 -: eine Handprothese;
- Figur 2-: eine Fingerprothese in Teilschnittansicht;
- Figur 3 -: eine Fingerprothese in Teilschnittansicht gemäß Schnittebene III aus Figur 2; sowie
- Figur 4 -: eine Fingerprothese in Querschnittsansicht gemäß Schnittebene IV der Figur 2.

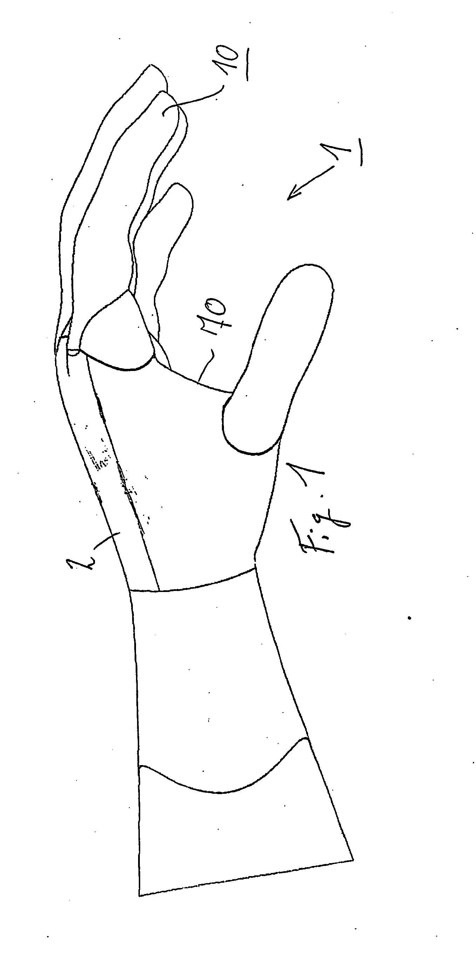

In der Figur 1 ist eine Handprothese 1 mit einem Handchassis 2 und daran gelenkig angeordneten Fingerprothesen 10 gezeigt. Über die gesamte Handprothese 1 ist ein Kunststoffüberzug 70 in einer Hautgestaltung aus einem elastischen Material in Handschuhform gezogen. Zumindest drei Finger, nämlich der Daumen, der Zeigefinger und der Mittelfinger sind gelenkig und antreibbar an dem Chassis 2 gelagert, da mit diesen drei Fingern ein Großteil der alltäglichen Greifverrichtungen ausgeführt werden kann.

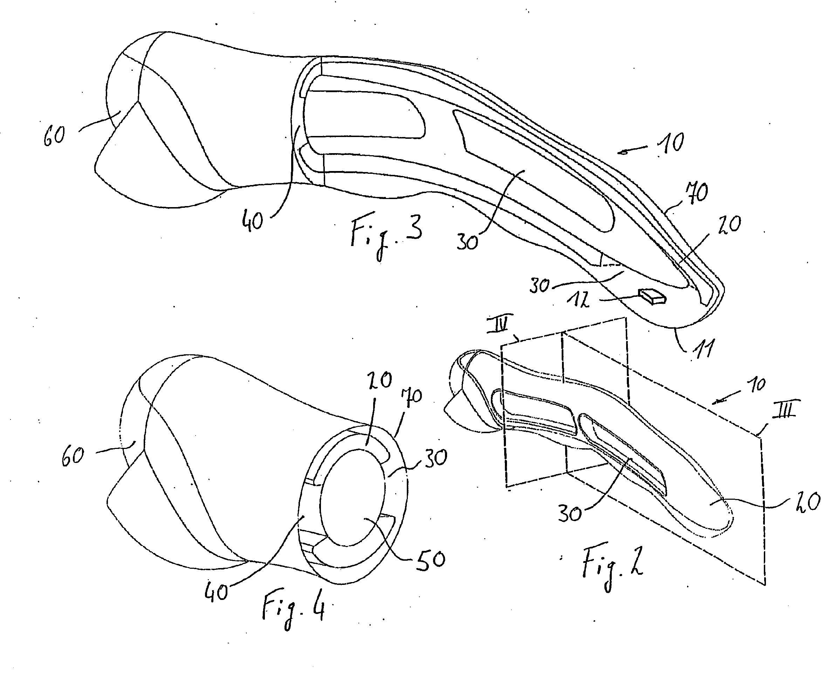

In der Figur 2 ist der Aufbau einer Fingerprothese 10 mit einem Grundelement 20 gezeigt, das bereits eine Grundkontur eines natürlichen Fingers aufweist. Das Grundelement 20 ist aus einem formstabilen Werkstoff, insbesondere Kunststoff oder Verbundmaterial aufgebaut und weist Ausnehmungen 30 in Bereichen auf, die den Fingergliedern und der Fingerspitze entsprechen. Diese Ausnehmungen 30 sind an denjenigen Stellen angeordnet, an denen Weichteilgewebe bei natürlichen Fingern vorhanden ist. Die Struktur des Grundelementes 20 ist dabei so ausgebildet, dass die Ausnehmungen 30 die mechanische Stabilität nicht beeinträchtigen, so dass die auf die Fingerprothese 10 wirkenden Kräfte weiterhin sicher aufgenommen und übertragen werden können. Vorliegend sind die Ausnehmungen 30 senkrecht zu einer Bewegungsebene der Fingerprothese 10 ausgebildet.

In der Figur 3 ist der Aufbau der Fingerprothese 10 in einer Teilschnittdarstellung gezeigt. Das Grundelement 20 erstreckt sich dabei im Wesentlichen über die gesamte Fingerlänge, wobei die Ausnehmungen 30 als Durchbrüche ausgebildet sind, durch die ein Elastomerwerkstoff 40 hindurchtreten kann, so dass in bestimmten Bereichen das als Hohlkörper ausgebildete Grundelement 20 von dem Elastomerwerkstoff 40 durchdrungen werden kann. Im Bereich der Fingerspitze 11 ist eine relativ große Ausnehmung oder ein relativ großer Bereich mit dem Elastomerwerkstoff 40 ausgefüllt, entsprechend dem Aufbau eines natürlichen Fingers. In der Fingerspitze ist ein Griffkraftsensor 12 eingebettet, um eine Rückmeldung an eine Steuereinheit zur Steuerung der aufzubringenden Greifkräfte zu geben. Alternativ oder ergänzend zu einem Griffkraftsensor 12 können ein Temperatursensor, ein Beschleunigungssensor oder ein Rutschsensor, der eine Relativbewegung zwischen der Fingerprothese und einem gegriffenen Gegenstand ermittelt, angeordnet sein. Der Rutschsensor kann optisch arbeiten, wobei die Anordnung der Sensoren nicht auf die Fingerspitze beschränkt ist, sondern diese an geeigneter Stelle überall in der Fingerprothese befestigt sein können.

In der Fingerprothese 10 ist eine Lagerung 60 im Bereich des Fingergrundgelenkes angeordnet, die eine starre Achse in einer weichen Lagerung aufweist, um eine seitliche Verlagerbarkeit senkrecht zur Drehachse um das Fingergrundgelenk zu ermöglichen. Eine solche Verlagerbarkeit schont die Komponenten und die Lagerung bei Belastungen, die beispielsweise bei einem Anstoßen gegen einen festen Gegenstand auftreten können. Neben einer starren und einteiligen Ausgestaltung der Fingerprothese 10 ist es möglich, diese mit zumindest einem weiteren Gelenk neben dem Fingergrundgelenk vorzusehen.

In der Figur 4 in Querschnittsdarstellung ist zu erkennen, dass das Grundelement 20 als ein Hohlkörper ausgebildet ist, dessen Wandung mit Ausnehmungen 30 in Gestalt von Durchbrüchen versehen ist, die mit dem Elastomerwerkstoff 40 aufgefüllt sind. Innerhalb des Grundelements 20 kann ein Kanal 50 ausgebildet sein, der Antriebe, Kabel oder Sensoren aufnehmen kann. Alternativ kann der Hohlraum mit dem Elastomerwerkstoff 40 ausgefüllt sein, wie in Figur 3 im Bereich der Fingerspitze dargestellt ist. Das Grundelement 20 wird vorteilhafterweise in einem 2K-Spritzgießverfahren zusammen mit dem Elastormerwerkstoff 40 verarbeitet, so dass die Kontur der Fingerprothese nach Beendigung des Spritzgießvorganges vollständig vorliegt. Auf die aufwendige Fertigung eines die Kontur bildenden Kunststoffüberzuges kann verzichtet werden.

Sowohl über eine einzelne Fingerprothese 10 als auch über die gesamte Handprothese 1 kann ein elastischer Kunststoffüberzug, der sehr dünnwandig ausgebildet sein kann, gezogen werden, um eine Hautstrukturierung und eine natürliche Farbgestaltung der Fingerprothese 10 zu ermöglichen.

Durch die erfindungsgemäße Fingerprothese 10 ist die Prothese an den richtigen Stellen hart und weich, gleichzeitig ist die Fingerprothese 10 sehr leicht und einfach herzustellen und hat durch das Anformen des Elastomerwerkstoffes 40 an die kraftübertragende Struktur des Grundelementes 20 eine der natürlichen Erscheinungsformen angenäherte Funktion, Haptik und Optik. Sofern nicht sämtliche Fingerprothesen 10 bei einer Handprothese 1 angetrieben sind, können die nicht angetriebenen Finger vollständig aus einem Elastomer ausgebildet sein, da diese in der Regel keine Kräfte übertragen müssen.

## Patentansprüche

1. Fingerprothese mit einem Grundelement, das zur Aufnahme von Kräften und Momenten ausgebildet und mit einem elastischen Überzug zur Erzielung einer natürlichen Optik versehbar ist, **dadurch gekennzeichnet, dass** das Grundelement (20) als ein formstabiler Kern ausgebildet ist, der an mit Weichteilstellen an natürlichen Fingern korrespondierenden Stellen Ausnehmungen (30) aufweist, die mit einem Elastomerwerkstoff (40) unter Bildung einer natürlichen Fingerkontur aufgefüllt sind.

2. Fingerprothese nach Anspruch 1, **dadurch gekennzeichnet, dass** das Grundelement (20) als Hohlkörper ausgebildet ist.

3. Fingerprothese nach Anspruch 1 oder 2, **dadurch gekennzeichnet, dass** das Grundelement (20) aus einem Kunststoff, insbesondere einem Thermoplasten oder einem Verbundwerkstoff; oder Metall, insbesondere Leichtmetall aufgebaut ist.

4. Fingerprothese nach einem der voranstehenden Ansprüche, **dadurch gekennzeichnet, dass** das Grundelement (20) und der Elastomerwerkstoff (40) in einem Zwei-Komponenten-Spritzgießverfahren gemeinsam verarbeitet sind.

5. Fingerprothese nach einem der voranstehenden Ansprüche, **dadurch gekennzeichnet, dass** das Grundelement (20) eine durchbrochene Struktur aufweist.

6. Fingerprothese nach einem der voranstehenden Ansprüche, **dadurch gekennzeichnet, dass** in dem Grundelement (20) und/oder dem Elastomerwerkstoff (40) Kanäle (50) und Aufnahmen für Kabel und Sensoren ausgebildet sind.

7. Fingerprothese nach einem der voranstehenden Ansprüche, **dadurch gekennzeichnet, dass** an dem Grundelement (20) eine Lagerung (60) für eine Gelenkachse ausgebildet ist.

8. Fingerprothese nach Anspruch 7, **dadurch gekennzeichnet, dass** die Lagerung (60) elastisch ausgebildet ist.

9. Fingerprothese nach Anspruch 7 oder 8, **dadurch gekennzeichnet, dass** im Bereich des Fingergrundgelenkes Einlegeteile aus Metall zu Verstärkung der Lagerung (60) angeordnet sind.

10. Fingerprothese nach einem der voranstehenden Ansprüche, **dadurch gekennzeichnet, dass** in der Fingerprothese (10), insbesondere im Bereich der Fingerspitze (11) ein Sensor (12) angeordnet ist.

11. Fingerprothese nach einem der voranstehenden Ansprüche, **dadurch gekennzeichnet, dass** über das Grundelement (20) und den Elastomerwerkstoff (40) ein auswechselbarer Kunststoffüberzug (70) in einer Hautgestaltung angeordnet ist.

12. Fingerprothese nach einem der voranstehenden Ansprüche, **dadurch gekennzeichnet, dass** der Elastomerwerkstoff (40) auf der Außenseite des Grundelementes (20) aufgebracht ist und dieses zumindest bereichsweise umschließt.

13. Handprothese (1) mit einem Chassis (2) und zumindest einer Fingerprothese (10) nach einem der voranstehenden Ansprüche.

14. Handprothese nach Anspruch 13, **dadurch gekennzeichnet, dass** zumindest eine Fingerprothese (10) gelenkig und/oder elastisch an dem Chassis (2) gelagert ist.

15. Handprothese nach Anspruch 13 oder 14, **dadurch gekennzeichnet, dass** über das Chassis (2) und die Fingerprothese (10) ein auswechselbarer Kunststoffüberzug (70) in einer Hautgestaltung angeordnet ist.

## Claims

1. Finger prosthesis comprising a base element, which is formed to receive forces and torques and can be provided with an elastic covering to achieve a natural appearance, **characterized in that** the base element (20) is formed as a dimensionally stable core which, in places which correspond to the soft parts of natural fingers, has clearances (30) that are filled with an elastomer material (40), thereby producing a natural contour of the finger.

2. Finger prosthesis according to Claim 1, **characterized in that** the base element (20) is formed as a hollow body.

3. Finger prosthesis according to Claim 1 or 2, **characterized in that** the base element (20) is made of a plastic, in particular a thermoplastic or a composite material, or metal, in particular lightweight metal.

4. Finger prosthesis according to one of the preceding claims, **characterized in that** the base element (20) and the elastomer material (40) are processed together in a two-component injection-moulding process.

5. Finger prosthesis according to one of the preceding claims, **characterized in that** the base element (20) has a broken structure.

6. Finger prosthesis according to one of the preceding claims, **characterized in that** channels (50) and holders for cables and sensors are formed in the base element (20) and/or the elastomer material (40).

7. Finger prosthesis according to one of the preceding claims, **characterized in that** a mounting (60) for an axial spindle of a joint is formed on the base element (20).

8. Finger prosthesis according to Claim 7, **characterized in that** the mounting (60) is elastically formed.

9. Finger prosthesis according to Claim 7 or 8, **characterized in that** metal inserts are arranged in the region of the metacarpophalangeal joint to reinforce the mounting (60).

10. Finger prosthesis according to one of the preceding claims, **characterized in that** a sensor (12) is arranged in the finger prosthesis (10), in particular in the region of the fingertip (11).

11. Finger prosthesis according to one of the preceding claims, **characterized in that** an exchangeable plastic covering (70) designed in the form of skin is arranged over the base element (20) and the elastomer material (40).

12. Finger prosthesis according to one of the preceding claims, **characterized in that** the elastomer material (40) is applied on the outer side of the base element (20) and encloses the latter at least in certain regions.

13. Hand prosthesis (1) comprising a chassis (2) and at least one finger prosthesis (10) according to one of the preceding claims.

14. Hand prosthesis according to Claim 13, **characterized in that** at least one finger prosthesis (10) is articulated and/or elastically coupled to the chassis (2).

15. Hand prosthesis according to Claim 13 or 14, **characterized in that** an exchangeable plastic covering (70) designed in the form of skin is arranged over the chassis (2) and the finger prosthesis (10).

## Revendications

1. Prothèse de doigt avec un élément de base, qui est agencé pour soutenir des forces et des moments et peut être doté d'un revêtement élastique pour l'obtention d'un aspect naturel, **caractérisé en ce que** l'élément de base (20) est réalisé sous la forme d'un noyau de forme stable, qui présente à des emplacements correspondant à des emplacements de parties molles pour des doigts naturels des évidements (30) qui sont remplis d'un matériau élastomère (40) à la forme du contour d'un doigt naturel.

2. Prothèse de doigt selon la revendication 1, **caractérisé en ce que** l'élément de base (20) est réalisé sous la forme d'un corps creux.

3. Prothèse de doigt selon l'une quelconque des revendications 1 ou 2, **caractérisé en ce que** l'élément de base (20) est construit en matière plastique, en particulier un thermoplastique ou un matériau composite ; ou en métal, en particulier un alliage léger.

4. Prothèse de doigt selon l'une quelconque des revendications précédentes, **caractérisé en ce que** l'élément de base (20) et le matériau élastomère (40) sont fabriqués ensemble par moulage par injection bi-matériaux.

5. Prothèse de doigt selon l'une quelconque des revendications précédentes, **caractérisé en ce que** l'élément de base (20) présente une structure ajourée.

6. Prothèse de doigt selon l'une quelconque des revendications précédentes, **caractérisé en ce que** des canaux (50) et logements pour câbles et capteurs sont réalisés dans l'élément de base (20) et/ou le matériau élastomère (40).

7. Prothèse de doigt selon l'une quelconque des revendications précédentes, **caractérisé en ce qu'**un coussinet (60) pour un axe d'articulation est formé dans l'élément de base (20).

8. Prothèse de doigt selon la revendication 7, **caractérisé en ce que** le coussinet (60) est réalisé sous une forme élastique.

9. Prothèse de doigt selon l'une quelconque des revendications 7 ou 8, **caractérisé en ce que** des pièces d'insertion en métal pour renforcer le coussinet (60) sont disposées dans la région de l'articulation de base du doigt.

10. Prothèse de doigt selon l'une quelconque des revendications précédentes, **caractérisé en ce qu'**un capteur (12) est disposé dans ladite prothèse de doigt (10), en particulier dans la région de l'extrémité (11) du doigt.

11. Prothèse de doigt selon l'une quelconque des revendications précédentes, **caractérisé en ce qu'**un revêtement plastique (70) interchangeable est disposé comme une peau au dessus de l'élément de base (20) et du matériau élastomère (40).

12. Prothèse de doigt selon l'une quelconque des revendications précédentes, **caractérisé en ce que** le matériau élastomère (40) vient par dessus l'extérieur de l'élément de base (20) et entoure celui-ci au moins partiellement.

13. Prothèse de main avec un châssis (2) et au moins une prothèse de doigt (10) selon l'une quelconque des revendications précédentes.

14. Prothèse de main selon la revendication 13, **caractérisé en ce qu'**au moins une prothèse de doigt (10) est montée de façon articulée ou élastique dur le châssis (2).

15. Prothèse de main selon l'une quelconque des revendications 13 ou 14, **caractérisé en ce qu'**un revêtement plastique (70) interchangeable est disposé comme une peau au dessus du châssis (2) et de la prothèse de doigt (10).
